# NEW EUROPEAN PATENT SPECIFICATION

(11) **EP 2 346 356 B2**
(45) Date of publication and mention of the opposition decision: **07.08.2019**
(45) Mention of the grant of the patent: 30.04.2014
(21) Application number: 09743961.6
(22) Date of filing: 23.10.2009
(51) Int. Cl.: A23L 1/29, A23L 1/30

(54) **LIQUID HIGH-FAT PROTEIN COMPOSITION**
FLÜSSIGE PROTEINZUSAMMENSETZUNG MIT HOHEM FETTGEHALT
COMPOSITION LIQUIDE DE PROTÉINES À HAUTE TENEUR EN MATIÈRES GRASSES

(30) Priority: 24.10.2008 WO PCT/NL2008/050669
(43) Date of publication of application: 27.07.2011
(73) Proprietor: N.V. Nutricia, 2712 HM Zoetermeer (NL)
(72) Inventor: HUISMAN, Ingmar Harald, NL-2627 AD Delft (NL); KIERS, Wynette Hermina Agnes, NL-6671 GB Zetten (NL); SLIWINSKI, Edward Lucian, NL-5342 EM Oss (NL); HOUGEE, Sander, NL-3972 XZ Driebergen-Rijsenburg (NL); MOLENAAR-GROENENDAAL, Marike Joanna Bernadette, NL-6942 TK Didam (NL)
(74) Representative: Nederlandsch Octrooibureau
(86) International application number: PCT/NL2009/050644
(87) International publication number: WO 2010/047597

(56) References cited:
- DE-A1- 4 304 394
- US-A- 4 690 820
- US-A- 4 921 877
- US-A- 5 470 839
- US-A- 5 571 553
- US-A1- 2002 192 354

## Description

### FIELD OF THE INVENTION

The invention relates to a shelf-stable liquid aqueous nutritional composition comprising at least non-micellar casein, a high amount of fat, and a heat stabilisation system. Such nutritional composition is suitable for persons with a (partially) functional gastrointestinal tract, who are unwilling and/or unable to consume sufficient quantities of conventional food to meet their nutritional requirements. The nutritional composition according to the invention is especially suitable for malnourished persons or persons at risk of becoming malnourished, and in need of liquid oral nutrition.

### BACKGROUND OF THE INVENTION

### Clinical problem

High-energy liquid nutritional compositions or supplements are a last resort for severely-ill persons that are hardly capable of orally taking in nutrition and/or fluids to maintain an adequate nutritional level.

Typically, such persons may be, for example, persons with various oesophagal cancers, strokes, neuromuscular dystrophy, Parkinson's disease, anorexia, renal failure, persons in cancer therapy which causes nausea and vomiting, resulting in a more or less long interruption of a person's normal eating habits, or persons with a restricted fluid intake. Such persons are at risk of becoming malnourished and use can be made of, for example enteral tube nutrition, intravenous nutrition or concentrated oral nutrition, such as high-energy, high-fat, or high-protein nutrition.

This invention concerns a shelf-stable high-fat high-energy liquid aqueous nutritional composition, preferably as a supplement. Furthermore, said shelf-stable high-fat high-energy liquid aqueous nutritional composition or supplement can also been envisioned for metabolically stressed people, elderly, or any person in need for the management of conditions requiring high-energy and/or high-fat nutrition.

### Technical problem

A number of high-fat high-energy liquid compositions have been disclosed in the literature.

US 2002/0192354 (Fuchs et al., 2002) discloses a calorically dense liquid oral supplement, including caseinate or acid casein and soy protein that provide at least 15 % of the total caloric content of the product. The product has a caloric density of at least 2.25 calories per ml. Table 1 discloses a composition containing 13 g fat per 100 ml.

US 4 690 820 (Vlado Simko, 1987) discloses an aqueous 3.6 kcal/ml high-energy high-fat dietary formula comprising 30 g/100 ml of fat (vegetable oil) and 6 g/100 ml of calcium caseinate, suitable for critically-ill persons.

US 4,921,877 (Cashmere et al., 1990) discloses nutritionally complete enteral formula for the dietary management of patients with hyperglycemia, such as those with diabetes mellitus or stress-induced hyperglycemia. The formula comprises about 33En % of carbohydrates, about 50 En% of fat and about 17 En%of protein.

DE 43 04 394 discloses an enteral nutritional composition having a specific fatty acid composition. It typically comprises about 40 - 65 En% fat. In the examples, the fat amounts are lower than 9 g per 100 ml.

US 5,470,839 discloses a nutritional supplement for a diabetic composition. According to the example, fat amounts to about 5 g per 100 ml.

US 5,571,553 concerns a food product comprised of a partially hydrogenated structured lipid in an amount of 40 - 80 %, and 20 - 60 % non-fat components. The preferred food is a candy bar.

A product, similar as described in the above patent publication, Pro-Cal Shot™ (Vitaflo International Ltd) is marketed as a 3.34 kcal/ml liquid high-calorie high-fat aqueous emulsion comprising 28.2 g/100 ml of fat and 6.7 g of protein.

WO 03/017774 (ISIS Innovation Ltd, 2003) discloses an aqueous palatable high-fat aqueous emulsion comprising 50 g/100 ml of oil and less than 1 g/100 ml of protein as part of a cocoa powder (Example 1), suitable for easy ingestion. Said composition is not sterilised.

Furthermore, several products have been described that deliver high amounts of fat, such as Calogen (SHS), and Microlipid (Nestle), both a 4.5 kcal/ml 50 % fat emulsion, however these products do not substantially contain a protein source.

Also, Resource Benecalorie (Nestle) is a 7 kcal/ml calorically-dense fat/protein supplement, containing 91 weight% of fat and 9 weight% of casein, however not containing any water.

Ordinary cream, derived from skimming milk, has generally a fat content of 40 g/100 ml, but need to be standardized to a fat content of 15 to 25 g/100 ml before being sterilized. Adding additional fat, such as disclosed in EP 1 815 748 A1 (Campina, 2007), even with a thickener as a processing aid, is still limited to a maximum fat content of 30 g/100 ml.

One of the technical problems associated with nutritional compositions comprising protein and an amount of fat above 30 g/100 ml is the poor heat stability. To serve and qualify as a commercial nutritional product, a nutritional product, as well as the product according to the invention should be able to withstand sterilisation to ensure a certain microbiological quality. To further ensure a long shelf live, the product should be shelf-stable, meaning that no sedimentation, segregation, aggregation, flocculation or gelation of the individual components should occur after the heat treatment.

Another problem with an aqueous high-fat formulation is the increased risk of reversal of the emulsion with increasing fat concentration, i.e. from oil-in-water to water-in-oil emulsions.

The technical problem is further complicated when the nutritional composition according to the invention is enriched with minerals, trace elements and vitamins, such as calcium and vitamin D, or should have a Ca:P-ratio above 1, as these components tend to react with the other ingredients, leading to sedimentation.

In the prior art, said problems have been circumvented, for example, by providing dry powder formulations comprising up to 39 weight% of fat and free amino acids (EP 925 726 A1 (Pellico)).

### SUMMARY OF THE INVENTION

The inventors have now succeeded in providing a shelf-stable liquid aqueous nutritional composition comprising at least non-micellar casein, having more than 30 g fat per 100 ml of said liquid aqueous nutritional composition, and a heat stabilisation system.

In one embodiment, the present invention provides a shelf-stable liquid aqueous nutritional composition comprising at least non-micellar casein, having a fat content between 35 and 50 g per 100 ml of said liquid aqueous nutritional composition, said composition comprising a heat stabilisation system.

In another embodiment, the present invention provides a shelf-stable liquid aqueous nutritional composition comprising at least non-micellar casein, having a fat content of about 40 g per 100 ml of said liquid aqueous nutritional composition, said composition comprising a heat stabilisation system.

In another embodiment, the present invention provides a shelf-stable liquid aqueous nutritional composition comprising at least non-micellar casein, having a fat content of more than 30 g per 100 ml, preferably a fat content between 35 and 50 g per 100 ml, more preferably having a fat content of about 40 g per 100 ml, wherein the non-micellar casein content is at least 1 g per 100 ml, wherein all concentrations are calculated based on the total liquid aqueous nutritional composition. The composition comprises a heat stabilisation system.

In another embodiment, the present invention provides a shelf-stable liquid aqueous nutritional composition comprising at least non-micellar casein, having a fat content of more than 30 g per 100 ml, preferably a fat content between 35 and 50 g per 100 ml, more preferably having a fat content of about 40 g per 100 ml, wherein the non-micellar casein content ranges between 4 and 10 g per 100 ml, wherein all concentrations are calculated based on the total liquid aqueous nutritional composition. The composition comprises a heat stabilisation system.

In another embodiment, the present invention provides a shelf-stable liquid aqueous nutritional composition comprising at least non-micellar casein, having a fat content of about 40 g per 100 ml, wherein the non-micellar casein content is about 5 g per 100 ml, wherein all concentrations are calculated based on the total liquid aqueous nutritional composition. The composition comprises a heat stabilisation system.

Additionally or alternatively, the nutritional composition according to the invention may contain digestible carbohydrates, minerals, trace elements and vitamins in amounts sufficient to fulfil the recommended daily allowance.

### DETAILED DESCRIPTION OF THE INVENTION

The invention relates to a shelf-stable liquid aqueous nutritional composition comprising at least a non-micellar casein, more than 30 g fat per 100 ml of said composition, and comprising a heat stabilisation system.

### Fat

The shelf-stable liquid aqueous nutritional composition according to the invention is directed to compositions comprising more than 30 g fat per 100 ml of said composition. It is commonly known that 30 g per 100 ml is currently a practical limit in the production of said shelf-stable liquid aqueous nutritional compositions with a high-fat content.

With regard to the type of fat, a wide choice is possible, as long as the fat is of food quality.

The fat may include medium chain triglycerides (MCT, mainly 8 to 10 carbon atoms long), long chain triglycerides (LCT) or any combination of the two types.

MCTs are beneficial because they are easily absorbed and metabolized. Moreover, the use of MCTs will reduce the risk of nutrient malabsorption.

LCT sources, such as rapeseed oil, more in particular rapeseed oil low in erucic acid, sunflower oil, corn oil, palm kernel fat, coconut fat, palm oil, or mixtures thereof are preferred because they provide more energy per unit of fat.

In one embodiment, the fat is a liquid fat, i.e. an oil.

In one embodiment, the fat comprises 30 to 60 weight% of animal or algal fat, 40 to 70 weight% of vegetable fat and optionally 0 to 20 weight% of MCTs based on total fat of the nutritional composition according to the invention. The animal fat preferably comprises a low amount of milk fat, i.e. lower than 6 weight%, especially lower than 3 weight%. In particular, a mixture of corn oil, egg oil, and/or canola oil and specific amounts of marine oil are used. Egg oils, fish oils and algal oils are a preferred source of non-vegetable fats. Marine oils containing DHA and/or EPA are preferably present in the nutritional composition according to the invention in an amount lower than 25 weight%, preferably lower than 15 weight% of the fat for obtaining a maximum health effect, such as, for instance, the prevention of cardiovascular risks. The amount of EPA ranges preferably between 4 weight% and 15 weight%, more preferably between 8 weight% and 13 weight% of the fat.

### Non-micellar casein

The liquid nutritional composition according to the invention comprises a non-micellar casein. The finding that a non-micellar casein is essential in producing a heat-stable formulation according to the invention is very surprising. It is known that caseinate acts as an emulsifier in aqueous fat emulsions, such as cream. But it is also known and it has been disclosed that the heat stability of cream deteriorates when increasing amounts of casein are covering the oil-water interface of the fat globules. (Walstra et al. Dairy Science and Technology, Second Ed. CRC Press - Taylor & Francis Group, Boca Raton, 2006, page 448).

The inventors have now found that a small amount of a non-micellar casein improves the heat stability of the liquid nutritional composition according to the invention. The amount of non-micellar casein may be routinely chosen by the skilled person and depends on the amount of other ingredients. Preferably, the amount of non-micellar casein in the nutritional composition according to the invention is at least 1 g per 100 ml of said composition. In another embodiment the amount of non-micellar casein in the nutritional composition according to the invention ranges between 1 and 10 g per 100 ml of said composition, preferably between 1 and 6 g per 100 ml of said composition.

Preferably, the non-micellar casein is sodium caseinate, potassium caseinate or a mixture thereof. When the nutritional composition further comprises a micellar casein, such as calcium caseinate, the composition should contain at least 20 to 40 weight%, preferably 25-35 weight% of non-micellar casein relative to the total amount of micllear and non-micellar casein/caseinate in the nutritional composition according to the invention.

The nutritional composition according to the invention may beneficially comprise further emulsifiers. An example of commonly known emulsifiers are phospholipids. Preferably, the nutritional composition according to the invention comprises 0.1 to 50 weight% phospholipids, based on the total weight of the lipids, more preferably 0.5 - 20 weight%, more preferably between 1 and 5 weight%, based on the total weight of the lipids. Preferably, the nutritional composition according to the invention comprises a phospholipid selected from the group of phosphatidyl choline, phosphatidyl ethanolamine, phosphatidyl serine and phosphatidyl inositol, or a mixture thereof. Apart from its emulsifier function, phospholipids may beneficially improve membrane function, thereby enabling an improved functioning of the different parts of the brain that play a (main) role in the ability to perform daily activities, such as in elderly persons. A common source of phospholipids is soy lecithin.

### Protein

The nutritional composition according to the invention optionally comprises at least a second protein other than non-micellar casein. In one embodiment, the nutritional composition according to the invention comprises as the second protein a milk protein, selected from the group of intact whey protein, whey protein hydrolysate (WPH), calcium caseinate, micellar casein (MCI) and mixtures thereof. In another embodiment, the nutritional composition according to the invention comprises as the second protein a vegetable protein, preferably selected from the group of pea, wheat, rice, potato, soy, corn and mixtures thereof. In another embodiment, the nutritional composition according to the invention comprises a mixture of milk protein and vegetable protein.

The second protein may be intact protein, hydrolysed protein, partially hydrolysed protein or mixtures thereof.

Preferably, the nutritional composition according to the invention comprises between 0.1 and 10 g of the second protein per 100 ml of said composition, more preferably between 0.1 and 8 g per 100 ml of said composition, still more preferably between 0.1 and 6 g per 100 ml relative to the total volume of the nutritional composition according to the invention.

The inventors have worked and demonstrated the invention using two common protein systems, a protein system comprising micellar casein (MCI), and a protein system comprising whey protein hydrolysate (WPH). Preferably, the second protein is selected from the group of whey protein hydrolysate (WPH), micellar casein (MCI) and mixtures thereof.

### Heat stabilisation system

In order to regulate the heat stability, the nutritional composition according to the invention includes a heat stabilisation system. With the wording "heat stabilisation system" is meant any compound or mixture of compounds, of organic or inorganic nature, that prevents the nutritional composition of the invention to sediment, segregate, aggregate, flocculate, gel or deteriorate in any other way upon sterilization such that a shelf-stable nutritional composition cannot be obtained. The heat stabilisation system is particularly preferred if the composition comprises as a second protein a milk protein selected from the group of intact whey protein, whey protein hydrolysate (WPH), calcium caseinate, micellar casein (MCI), and mixtures thereof. Particularly these proteins may require heat stabilisation when subjected to a heat treatment, such as a pasteurisation and/or sterilisation step. The heat stabilisation system is also preferably used when amounts of Ca-ions need to be added to the nutritional composition, as may be the case when a shelf-stable liquid aqueous nutritional composition is to be obtained with added amounts of calcium to achieve a (more) complete nutrition, The heat stabilisation system is preferably used when amounts of calcium are present in the shelf-stable liquid aqueous nutritional composition over 10 mg, such as more than 20, 30, 40, 50, 60, 70, 80, 90, 100, 110, 120, 130, 140, 150, 160, 170, 180, 190 or more than 200 mg Ca per 100 ml.

Although many heat stabilisation systems may be envisioned, the inventors have surprisingly found that a heat stabilisation system comprising potassium citrate (also called tripotassium citrate), potassium dihydrogen phosphate or mixtures thereof, is a preferred choice. More in particular, in case the second protein mainly comprises micellar casein, preferably, the heat stabilisation system mainly comprises potassium citrate. More in particular, in case the second protein mainly comprises whey protein hydrolysate, preferably, the heat stabilisation system mainly comprises potassium dihydrogen phosphate. Surprisingly, and although the need for a heat-stabilised high-fat nutritional composition is a long-felt need, such heat stabilisation system has not been described for a formulation with a high-fat content. Without being bound by theory, the inventors assume that free calcium, present in the formulation and responsible for, for example gelation during the heat treatment, is effectively bound by the added phosphate or citrate. Phosphate is most effectively used in the case of micellar casein, as it does not extract such large amounts of Ca from the micelles as citrate would do, such that the micelles swell and give rise to both an increase of the viscosity and gelation. Furthermore, the Ca-phosphate complex precipitates on the micelles and remains in suspension, while a Ca-citrate complex would precipitate much easier. In the context of this invention, with "mainly" is meant more than 50 % by weight or by volume, more preferably more than 60 %, 70 %, 80 %, 90 %, 95 %, or 99 % by weight or by volume, and most preferably about 100 % by weight or by volume.

### Other stabilisers

Preferably, the nutritional composition according to the invention further comprises a precipitation stabiliser in order to prevent insoluble components, especially insoluble minerals, from precipitating. Common precipitation stabilisers may be selected from the group of cellulose, xanthan gum, gellan gum, alginate, guar gum, locust bean gum, gum karaya, gum tragacanth, carrageenan, and mixtures thereof. A preferred choice is a cellulose-based precipitation stabiliser selected from the group of high viscous cellulose and cellulose derivatives such as, for example, hydroxyethyl cellulose, hydroxypropyl methyl cellulose, carboxymethyl cellulose (CMC), microcrystalline cellulose (MCC) and methyl cellulose, and mixtures thereof. A most preferred choice is a mixture of carboxymethyl cellulose and microcrystalline cellulose (CMC/MCC).

### Digestible carbohydrates

Advantageously, the nutritional composition according to the invention optionally comprises a digestible carbohydrate. Although the bulk of the nutritional energy is provided by the fat, a digestible carbohydrate may be included in the nutritional composition according to the invention to increase the nutritional energy, and with an additional purpose, for example, to improve the taste, or to have an advantageous effect over and above the nutritional effects of the nutritional composition according to the invention. The nutritional composition according to the invention may comprise a digestible carbohydrate content between 1 and 50 gram per 100 ml, relative to the total volume of the nutritional composition according to the invention, preferably between 1 and 30 g per 100 ml, more preferably between 1 and 10 g per 100 ml, most preferably between 1 and 5 g per 100 ml, most preferably equal to about 5 g per 100 ml, relative to the total volume of the nutritional composition according to the invention. The nutritional composition according to the invention may encompass any source of carbohydrates that may be naturally-occurring, synthetic, or developed through the genetic manipulation of organisms. For example, sources of carbohydrates that are suitable for use in the nutritional composition according to the invention may include, but are not limited to, corn syrup solids, maltodextrin, sugars such as glucose, fructose, dextrose, lactose, galactose, isomaltulose, saccharides, sucrose, and maltose ; sugar alcohols, such as sorbitol, mannitol and xylitol ; syrups such as maltitol, corn syrup, rice syrup and high fructose corn syrup ; and any mixture thereof.

### Flavouring agent

The nutritional composition according to the invention may also contain a flavouring agent, either natural, artificial or a mixture thereof. The incorporation of a flavouring agent increases the palatability of the nutritional composition according to the invention, so facilitating its ingestion and imparting a pleasant taste to the nutritional composition. Although the nutritional composition according to the invention without a flavouring agent already tastes pleasant, in contrast to prior art high-fat nutritional compositions, adding a flavouring agent to impart a particular flavour may further improve the palatability, thus increasing patient compliance. Often used flavours are chocolate, vanilla, coffee, caramel, cinnamon, strawberry, lemon, peach, orange, forest fruits, e.a. The nutritional composition according to the invention may also contain an artificial sweetener, such as, for instance saccharin, aspartame and the like. Other embodiments will be clear to those skilled in the art.

### Vitamins, minerals and other nutritional components

The nutritional composition according to the invention may contain a variety of vitamins and minerals. When the nutritional composition is intended as complete nutrition, the nutritional composition according to the invention preferably includes at least 100 % of the United States Recommended Daily Allowance (USRDA) of vitamins and minerals in a one litre portion, more preferably at least 35 % of the United States Recommended Daily Allowance (USRDA) of vitamins and minerals in a 100 ml portion.

In one embodiment of the present invention, the composition according to the invention provides all necessary vitamins and minerals. For example, the composition according to the invention preferably provides 6 mg of zinc per 100 ml of the composition which is beneficial for tissue repair in a healing person. Preferably, the composition according to the invention (also) provides 25 mg of vitamin C per 100 ml of the composition to aid persons with more severe healing requirements. Further, preferably, the composition according to the invention (also) provides 2.25 mg iron per 100 ml of the composition. Iron is beneficial in maintaining bodily fluids as well as circulatory system functions in an elderly person.

In another embodiment of the present invention, the amount of divalent ions in the nutritional composition according to the invention ranges between 170 and 230 mg per 100 ml of said composition and preferably between 180 and 220 mg per 100 ml of said composition. Preferably, the amount of calcium ranges between 155 and 185 mg per 100 ml of said composition and preferably between 160 and 180 mg per 100 ml of said composition. The phosphorus content can be above 10 mg per g of protein, with a calcium to phosphorus weight ratio between 1.0 and 2.0, preferably between 1.1 and 1.7. Carnitin may advantageously be present in an amount of 8 to 1000 mg per 100 ml of said composition, preferably in an amount of 10 to 100 mg per 100 ml of said composition ; it may have the form of carnitin, alkyl carnitin, acyl carnation or mixtures thereof. Organic acids are preferably present at a level of between 0.1 to 0.6 g per 100 ml of said composition, especially between 0.25 g to 0.5 g per 100 ml of said composition. These acids include short fatty acids such as acetic acid, hydroxy acids such as lactic acid, gluconic acid, and preferably polyvalent hydroxy acids, such as malic acid and citric acid. In one embodiment of the present invention, the present composition also comprises citric acid.

In another embodiment of the present invention, the nutritional composition according to the invention is intended as a supplement ; hence it may contain the aforementioned vitamins, minerals and other nutritional components in a lower, or even higher, amount mentioned above.

### Non-digestible carbohydrates

The liquid enteral nutritional composition according to the invention may optionally be fortified with non-digestible carbohydrates (dietary fibres), such as fructo-oligosaccharides or inulin.

In one embodiment of the present invention, the nutritional composition according to the invention comprises 0.5 g to 12 g of non-digestible carbohydrates per 100 ml of said composition. The dietary fibres may include non-digestible oligosaccharides having a DP of 2 to 20, preferably 2 to 10. It is however preferred that the dietary fibers include non-digestible oligosaccharides having a DP of at least 3, which brings the definition of dietary fibers in line with EU Directive 90/496 EEC on nutrition labelling for foodstuffs that entered into force on 29 October 2008. More preferably, these oligosaccharides do not contain substantial amounts of saccharides outside these DP ranges, and they are soluble. These oligosaccharides may comprise fructo-oligosaccharides (FOS), trans-galacto-oligosaccharides (TOS), xylo-oligosaccharides (XOS), soy oligosaccharides, and the like. Optionally, also higher molecular weight compounds such as inulin, cellulose, resistant starch and the like may be incorporated in the composition according to the invention. The amount of insoluble non-digestible carbohydrate such as cellulose is preferably lower than 20 weight% of the dietary fibre fraction of the composition according to the invention, and/or below 0.4 g/100 ml. The amount of thickening polysaccharides such as carrageenans, xanthans, pectins, galactomannans and other high molecular weight (DP > 50) indigestible polysaccharides is preferably low, i.e. less than 20 % of the weight of the fibre fraction, or less than 1 g per 100 ml of said composition.

A preferred fibre component is an indigestible oligosaccharide with a chain length (DP) of 2 to 10, for example Fibersol® (resistant oligoglucose), in particular hydrogenated Fibersol®, or a mixture of oligosaccharides having a DP of 2 to 10, such as fructo-oligosaccharides or galacto-oligosaccharides, which may also contain a small amount of higher saccharides (i.e. with a DP of 11 to 20). Such oligosaccharides preferably comprise 50 weight% to 90 weight% of the fibre fraction, or 0.5 g/100 ml to 3 g/100 ml of the composition according to the invention. Other suitable fibre components include saccharides that have only partial digestibility.

In another embodiment of the present invention, the composition according to the invention may comprise a mixture of neutral and acid oligosaccharides as disclosed in WO 2005/039597 (N.V. Nutricia), which is incorporated herin by reference in its entirety. More in particular, the acid oligosaccharide has a degree of polymerisation (DP) between 1 and 5000, preferably between 1 and 1000, more preferably between 2 and 250, even more preferably between 2 and 50, most preferably between 2 and 10. If a mixture of acid oligosaccharides with different degrees of polymerisation is used, the average DP of the acid oligosaccharide mixture is preferably between 2 and 1000, more preferably between 3 and 250, even more preferably between 3 and 50. The acid oligosaccharide may be a homogeneous or heterogeneous carbohydrate. The acid oligosaccharides may be prepared from pectin, pectate, alginate, chondroitine, hyaluronic acids, heparine, heparane, bacterial carbohydrates, sialoglycans, fucoidan, fucooligosaccharides or carrageenan, and are preferably prepared from pectin or alginate. The acid oligosaccharides may be prepared by the methods described in WO 01/60378, which is hereby incorporated by reference. The acid oligosaccharide is preferably prepared from high methoxylated pectin, which is characterised by a degree of methoxylation above 50%. As used herein, "degree of methoxylation" (also referred to as DE or "degree of esterification") is intended to mean the extent to which free carboxylic acid groups contained in the polygalacturonic acid chain have been esterified (e.g. by methylation). The acid oligosaccharides are preferably characterised by a degree of methoxylation above 20%, preferably above 50 % even more preferably above 70%. Preferably the acid oligosaccharides have a degree of methylation above 20%, preferably above 50 % even more preferably above 70%. The acid oligosaccharide is preferably administered in an amount of between 10 mg and 100 gram per day, preferably between 100 mg and 50 grams per day, even more between 0.5 and 20 gram per day.

The term neutral oligosaccharides as used in the present invention refers to saccharides which have a degree of polymerisation of monose units exceeding 2, more preferably exceeding 3, even more preferably exceeding 4, most preferably exceeding 10, which are not or only partially digested in the intestine by the action of acids or digestive enzymes present in the human upper digestive tract (small intestine and stomach) but which are fermented by the human intestinal flora and preferably lack acidic groups. The neutral oligosaccharide is structurally (chemically) different from the acid oligosaccharide. The term neutral oligosaccharides as used in the present invention preferably refers to saccharides which have a degree of polymerisation of the oligosaccharide below 60 monose units, preferably below 40, even more preferably below 20, most preferably below 10. The term monose units refers to units having a closed ring structure, preferably hexose, e.g. the pyranose or furanose forms. The neutral oligosaccharide preferably comprises at least 90%, more preferably at least 95% monose units selected from the group consisting of mannose, arabinose, fructose, fucose, rhamnose, galactose, β-D-galactopyranose, ribose, glucose, xylose and derivatives thereof, calculated on the total number of monose units contained therein. Suitable neural oligosaccharides are preferably fermented by the gut flora. Preferably the oligosaccharide is selected from the group consisting of: cellobiose (4-O-β-D-glucopyranosyl-D-glucose), cellodextrins ((4-O-β-D-glucopyranosyl)ₙ-D-glucose), B-cyclodextrins (Cyclic molecules of α-1-4-linked D-glucose; α-cyclodextrin-hexamer, β-cyclodextrin-heptamer and γ-cyclodextrin-octamer), indigestible dextrin , gentiooligosaccharides (mixture of β-1-6 linked glucose residues, some 1-4 linkages), glucooligosaccharides (mixture of α-D-glucose), isomaltooligosaccharides (linear α-1-6 linked glucose residues with some 1-4 linkages), isomaltose (6-O-α-D-glucopyranosyl-D-glucose); isomaltriose (6-O-α-D-glucopyranosyl-(1-6)-α-D-glucopyranosyl-D-glucose), panose (6-O-α-D-glucopyranosyl-(1-6)-α-D-glucopyranosyl-(1-4)-D-glucose), leucrose (5-O-α-D-glucopyranosyl-D-fructopyranoside), palatinose or isomaltulose (6-O-α-D-glucopyranosyl-D-fructose), theanderose (O-α-D-glucopyranosyl-(1-6)-O-α-D-glucopyranosyl-(1-2)-B-D-fructofuranoside), D-agatose, D-*lyxo*-hexulose, lactosucrose (O-β-D-galactopyranosyl-(1-4)-O-α-D-glucopyranosyl-(1-2)-β-D-fructoranoside), α-galactooligosaccharides including raffinose, stachyose and other soy oligosaccharides (O-α-D-galactopyranosyl-(1-6)-α-D-glucopyranosyl-β-D-fructofuranoside), β-galactooligosaccharides or transgalacto-oligosaccharides (β-D-galactopyranosyl-(1-6)-[β-D-glucopyranosyl]ₙ-(1-4)α-D glucose), lactulose (4-O-β-D-galactopyranosyl-D-fructose), 4'-galatosyllactose (O-D-galactopyranosyl-(1-4)-O-β-D-glucopyranosyl-(1-4)-D-glucopyranose), synthetic galactooligosaccharide (neogalactobiose, isogalactobiose, galsucrose, isolactose I, II and III), fructans - Levan-type (β-D-(2→6)-fructofuranosyl)ₙ α-D-glucopyranoside), fructans - Inulin-type (β-D-((2→1)-fructofuranosyl)ₙ α-D-glucopyranoside), 1 f-β-fructofuranosylnystose (β-D-((2→1)-fructofuranosyl)ₙ B-D-fructofuranoside), xylooligosaccharides (B-D-((1→4)-xylose)ₙ, lafinose, lactosucrose and arabinooligosaccharides.

According to a further preferred embodiment the neutral oligosaccharide is selected from the group consisting of fructans, fructooligosaccharides, indigestible dextrins galactooligosaccharides (including transgalactooligosaccharides), xylooligosaccharides, arabinooligosaccharides, glucooligosaccharides, mannooligosaccharides, fucooligosaccharides and mixtures thereof. Most preferably, the neutral oligosaccharide is selected from the group consisting of fructooligosacchararides, galactooligosaccharides and transgalactooligosaccharides. Suitable oligosaccharides and their production methods are further described in Laere K.J.M. >Laere, K.J.M., Degradation of structurally different non-digestible oligosaccharides by intestinal bacteria: glycosylhydrolases of Bi. adolescentis. PhD-thesis (2000), Wageningen Agricultural University, Wageningen, The Netherlands), the entire content of which is hereby incorporated by reference. Transgalactooligosaccharides (TOS) are for example sold under the trademark Vivinal™ (Borculo Domo Ingredients, Netherlands). Indigestible dextrin, which may be produced by pyrolysis of corn starch, comprises α(1→4) and α(1→6) glucosidic bonds, as are present in the native starch, and contains 1→2 and 1→3 linkages and levoglucosan. Due to these structural characteristics, indigestible dextrin contains well-developed, branched particles that are partially hydrolysed by human digestive enzymes. Numerous other commercial sources of indigestible oligosaccharides are readily available and known to skilled person. For example, transgalactooligosaccharide is available from Yakult Honsha Co., Tokyo, Japan. Soybean oligosaccharide is available from Calpis Corporation distributed by Ajinomoto U.S.A. Inc., Teaneck, N.J.

In a further preferred embodiment the composition according to the invention comprises
an acid oligosaccharide with a DP between 2 and 250, prepared from pectin, alginate, and mixtures thereof; and a neutral oligosaccharide, selected from the group of fructans, fructooligosaccharides, indigestible dextrins, galactooligosaccharides including transgalactooligosaccharides, xylooligosaccharides, arabinooligosaccharides, glucooligosaccharides, mannooligosaccharides, fucooligosaccharides, and mixtures thereof.

In a further preferred embodiment the composition according to the invention comprises two chemically distinct neutral oligosaccharides. It was found that the administration of acid oligosaccharides combined with two chemically distinct neutral oligosaccharides provides an optimal synergistic immune stimulatory effect. Preferably the composition according to the invention comprises :
- an acid oligosaccharides as defined above ;
- a galactose-based neutral oligosaccharide (of which more than 50 % of the monose units are galactose units), preferably selected from the group consisting of galactooligosaccharide and transgalactooligosaccharide; and
- a fructose and/or glucose based neutral oligosaccharide (of which more than 50% of the monose units are fructose and/or glucose, preferably fructose units), preferably inulin, fructan and/or fructooligosaccharide, most preferably long chain fructooligosaccharide (with an average DP of 10 to 60).

The mixture of acid- and neutral oligosaccharides is preferably administered in an amount of between 10 mg and 100 gram per day, preferably between 100 mg and 25 grams per day, even more preferably between 0.5 and 20 gram per day.

### Viscosity and osmolarity

In one embodiment of the present invention, the viscosity of the liquid enteral nutritional composition is lower than 150 mPa.s, preferably lower than 125 mPa.s, most preferably lower than 100 mPa.s, determined at 20 °C at a shear rate of 100 s⁻¹. The viscosity may be determined using a rotational viscosity meter using a cone/plate geometry.

In one embodiment of the present invention, the osmolarity of the composition is preferably lower than 900 mOsm/l, more preferably lower than 800 mOsm/1, more preferable lower than 700 mOsm/l, more preferable lower than 600 mOsm/l.

In one embodiment, the osmolarity of the nutritional composition according to the invention is lower than 500 mOsm/l, which may be obtained due to the absence or low levels of carbohydrates, such as, for example lower than 5 g/100 ml.

In one embodiment of the present invention, the density of the composition ranges between 0.90 g/ml and 1.20 g/ml, between 0.95 g/ml and 1.15 g/ml, especially between 0.95 g/ml and 1.05 g/ml.

In one embodiment of the present invention, the density of the composition ranges between 1.05 g/ml and 1.20 g/ml, especially between 1.10 g/ml and 1.18 g/ml.

In one embodiment of the present invention, the composition has an energy content of more than 270 kcal/100 ml, preferably more than 300 kcal/100 ml, preferably more than 350 kcal/100 ml, most preferably 400 kcal/100 ml or more.

### Dosage unit

The liquid enteral nutritional composition may have the form of a food supplement, a so-called nutritional shot, for example to be used in addition to a non-medical food. In one embodiment of the present invention, a unit dosage comprises any amount of the liquid enteral nutritional composition according to the invention between 10 ml and 250 ml, the end values of this range included, preferably any amount between 25 ml and 200 ml, the end values of this range included, more preferably any amount between 50 ml and 150 ml, the end values of this range included, most preferably about 125 ml. For example, a person receiving 25-50 ml unit dosages can be given 5-10 unit dosages per day to provide nutritional support using a liquid enteral nutritional composition according to the invention of 400 kcal/100 ml. Alternatively, a person receiving 125 ml unit dosages can be given 4 or 5 or 6 or 7 or 8 unit dosages per day to provide nutritional support using a liquid enteral nutritional composition according to the invention of 400 kcal/100 ml. Such small dosage units are preferred because of better compliance.

The liquid enteral nutritional composition according to the invention may also have the form of a complete food, i.e. it can meet all nutritional needs of the user. Preferably, it is given in combination with extra amounts of protein as the protein content of the nutritional composition according to the invention is low. As such, it preferably is dosed at 1200 to 2500 kcal per day. The daily dosage amounts are given with respect to a daily energy supply of 2000 kcal to a healthy adult having a body weight of 70 kg. For persons of different condition and different body weight, the levels should be adapted accordingly. In general, it is accepted that the average daily energy intake of a human is about 2000 kcal.

The liquid enteral nutritional composition according to the invention can be in the form of multiple dosage units, for example from 2 (250 ml/unit) to 10 (50 ml/unit) per day for an energy supply of 2000 kcal/day using a liquid enteral nutritional composition according to the invention of 400 kcal/100 ml.
In the preferred embodiment of the present invention, the composition is provided in a ready to use liquid form and does not require reconstitution or mixing prior to use. The composition according to the invention can be tube fed or administered orally. For example, the composition according to the invention can be provided in a can, on a spike, or in a hangbag.

In one embodiment of the present invention, the composition according to the invention is packaged. The packaging may have any suitable form, for example a block-shaped carton (brick), for example to be emptied with a straw ; a carton or plastic beaker with removable cover ; a small-sized bottle for example for the 80 ml to 200 ml range, and small cups for example for the 10 ml to 30 ml range. Another suitable packaging mode is inclusion of small volumes of liquid (for example 10 ml to 20 ml) in edible solid or semi-solid hulls or capsules, for example gelatine-like coverings and the like.

### Method of preparation

The liquid enteral nutritional composition according to the invention may be prepared by first preparing a composition comprising the precipitation stabilizer, the casein source and optionally other proteins, the heat stabilisation system, and optionally carbohydrates and minerals. The proteins may be added by sequentially or simultaneously dissolving micellar casein in powder form and caseinate in powder form in water. It is also possible to use micellar casein in a wet form, directly prepared from milk. It may even be advantageous to prepare the micellar casein as a part of a continuous process to prepare the composition according to the invention. The latter may be done in the same production facility to prepare the composition according to the invention.

According to one embodiment, a first aqueous composition is prepared by dissolving the optional precipitation stabiliser in water, and subsequently adding the casein source, optional other proteins, carbohydrates and the emulsifier. If desired, a second aqueous composition may be prepared by dissolving a heat stabilisation system and optionally minerals in water and the first and second compositions are subsequently mixed with each other into a third aqueous composition. Optionally, a trace element mix may be prepared as a fourth aqueous composition and subsequently mixed with any one of the other aqueous compositions.

According to another embodiment, a first aqueous composition is prepared by dissolving the casein source, optional other proteins, carbohydrates and the emulsifier in water. A second aqueous composition may be prepared by dissolving the heat stabilisation system, precipitation stabiliser and/or minerals in water and the first and second composition are subsequently mixed with each other into a third aqueous composition. Optionally, a trace element mix may be prepared as a fourth aqueous composition and subsequently mixed with any one of the other aqueous compositions.

Next, the pH of third aqueous composition should be adjusted to about 6.5 and 7.2, using common pH adjustment agents, such as citric acid.

Next, the fat source is added to the third aqueous composition and the resulting composition is emulsified and subjected to pasteurisation. Typical pasteurisation times are 12 to 36 sec at 85 °C.

Optionally, other additives such as colorants, flavourings and vitamins may be added to the pasteurised solution.

Next, the pH of the resulting composition is adjusted to a final pH about 6.5 and 7.2, using common agents, such as citric acid, and the composition is sterilised. Typical sterilisation times are 4 to 8 minutes at 124 °C or 30 to 120 seconds at 132 °C. It is noticed that the sterilisation time is preferably relatively long, as the heat transport through the fat-rich composition is slower than through compositions comprising lower amounts of fat.

Finally, the resulting product may be packaged.

The following non-limiting examples illustrate the invention.

### EXAMPLES

The following compositions according to the invention have been prepared (Tables 1A, 1B1, 1B2, 1C, 1D and 2). The composition is produced in a manner as described above, is shelf-stable, has desirable organoleptic properties, has a very high nutrient density and is effective for a person in need thereof.

**Table 1A : Without optional protein**

| **Ingredient** | **Ex. A1** | **Ex. A2** | **Ex. A3** |
|---|---|---|---|
| **Energy content** (kcal/100 ml) | **400** | **400** | **400** |
| **Fat** (g/100 ml) | **41.50** | **41.40** | **41.50** |
| Blend of rapeseed/sunflower high oleic | 40.15 | 40.05 | 37.43 |
| Soy lecithin (emulsifier) | 1.35 | 1.35 | 1.35 |
| Fish oil | - | - | 2.72 |
| **Protein** (g/100 ml) | 1.7 | 1.9 | 1.7 |
| Non-micellar casein (Na-caseinate) | 1.7 | 1.7 | 1.7 |
| Vegetable protein (cacao source) | - | 0.2 | - |
| **Carbohydrates** (g/100 ml) | **4.80** | **5.06** | **4.00** |
| sugars | 3.54 | 3.64 | 3.54 |
| sucrose | 3.49 | 3.57 | 3.49 |
| maltose | 0.05 | 0.07 | 0.05 |
| polysaccharides (CMC/MCC) | 0.09 | 0.20 | 0.09 |
| organic acids | 1.07 | 1.04 | 1.07 |
| others | 0.10 | 0.18 | 0.10 |
| **Heat stabiliser** (g/100 ml) | | | |
| tripotassium citrate | 1.455 | 1.5 | 1.455 |
| **Minerals** (mg/100 ml) | | | |
| Sodium | 38 | 35 | 38 |
| Potassium | 535 | 535 | 535 |
| Calcium | 222 | 223 | 222 |
| Phosphorous | 189 | 288 | 189 |
| Magnesium | 89 | 89 | 89 |
| Chloride | 47 | 48 | 47 |
| **Flavour** | strawberry | chocolate | vanilla |

**Table 1B1 : Without optional protein and with dietary fiber**

| **Ingredient** | **Ex. B1** | **Ex. B2** | **Ex. B3** |
|---|---|---|---|
| **Energy content** (kcal/100 ml) | **400** | **400** | **400** |
| **Fat** (g/100 ml) | **41.50** | **41.50** | **41.50** |
| Blend of rapeseed/sunflower high oleic | 41.50 | 41.50 | 38.78 |
| Fish oil | - | - | 2.72 |
| **Protein** (g/100 ml) | | | |
| Non-micellar casein (Na-caseinate) | **1.7** | **1.7** | **1.7** |
| **Carbohydrates** (g/100 ml) | **4.80** | **4.80** | **4.80** |
| sugars | 3.54 | 3.54 | 3.54 |
| sucrose | 3.49 | 3.49 | 3.49 |
| maltose | 0.05 | 0.05 | 0.05 |
| polysaccharides (CMC/MCC) | 0.09 | 0.09 | 0.09 |
| organic acids | 1.07 | 1.07 | 1.07 |
| others | 0.10 | 0.10 | 0.10 |

| **Heat stabiliser** (g/100 ml) | | | |
|---|---|---|---|
| tripotassium citrate | 1.455 | 1.455 | 1.455 |
| monopotassium phosphate (KH₂PO₄) | - | - | - |
| **Non-digestible carbohydrates** (g/100 ml) | **9*** | **9*** | **9*** |
| GOS/long chain-FOS (9:1) | 9 | 7.5 | 7.5 |
| low viscosity pectin | - | 1.5 | 1.5 |

| **Minerals** (mg/100 ml) | | | |
|---|---|---|---|
| Sodium | 20 | 20 | 20 |
| Potassium | 20 | 20 | 20 |
| Calcium | 2 | 2 | 2 |
| Phosphorous | 14 | 14 | 14 |
| Magnesium | 4 | 4 | 4 |
| Chloride | 40 | 40 | 40 |
| * energy contribution fibers not taken into account | | | |

**Table 1B2 : With and without (Ex. B4, B7) heat stabilisation system**

| **Ingredient** | **Ex. B4** | **Ex. B5** | **Ex. B6** | **Ex. B7** | **Ex. B8** | **Ex. B9** |
|---|---|---|---|---|---|---|
| **Energy content** (kcal/100 ml) | **400** | **403** | **407** | **400** | **403** | **407** |
| **Fat** (g/100 ml) | **41** | **41** | **41** | **41** | **41** | **41** |
| **Protein** (g/100 ml) | 1.0 | 1.0 | 1.0 | 1.0 | 1.0 | 1.0 |
| Non-micellar casein (Na-caseinate) | 1.0 | 1.0 | 1.0 | 1.0 | 1.0 | 1.0 |
| **Carbohydrates** (g/100 ml) | **3.8** | **4.0** | **4.6** | **3.8** | **4.0** | **4.6** |
| organic acids | | 0.3 | 0.9 | | 0.3 | 0.9 |
| lactose | 1.9 | 1.9 | 1.9 | 1.9 | 1.9 | 1.9 |

| **Heat stabiliser** (g/100 ml) | | | | | | |
|---|---|---|---|---|---|---|
| tripotassium citrate monopotassium phosphate (KH₂PO₄) | 0.0 | 0.41 | 1.3 | 0.00 | 0.41 | 1.30 |
| **Non-digestible carbohydrates***** (g/100 ml) | **7.5** | **7.5** | **7.5** | **7.5** | **7.5** | **7.5** |
| GOS/long chain-FOS (9:1) | 6.3 | 6.3 | 6.3 | 6.3 | 6.3 | 6.3 |
| low viscosity pectin | 1.3 | 1.3 | 1.3 | 1.3 | 1.3 | 1.3 |

| **Minerals** (mg/100 ml) | | | | | | |
|---|---|---|---|---|---|---|
| Sodium | 24 | 24 | 24 | 57* | 57* | 57* |
| Potassium** | 63 | 208 | 535 | 63 | 208 | 535 |
| Calcium | 6 | 6 | 6 | 6 | 6 | 6 |
| Phosphorous | 8 | 8 | 8 | 8 | 8 | 8 |
| Magnesium | 1 | 1 | 1 | 1 | 1 | 1 |
| Chloride | 5 | 5 | 5 | 5 | 5 | 5 |

| **Vitamins** | | | | | | |
|---|---|---|---|---|---|---|
| Vitamin C (mg/100 ml) | | | | 166.7 | 166.7 | 166.7 |
| Vitamin D(µg/100 ml) | | | | 16.7 | 16.7 | 16.7 |
| Vitamin E(IU/100 ml) | | | | 149.0 | 149.0 | 149.0 |
| Selenium (µg/100 ml) | | | | 166.7 | 166.7 | 166.7 |
| **Ingredient** | **Ex. B4** | **Ex. B5** | **Ex. B6** | **Ex. B7** | **Ex. B8** | **Ex. B9** |
| Zinc (mg/100 ml) | | | | 8.3 | 8.3 | 8.3 |
| * The additional sodium originates from sodium ascorbate (Vit C); | | | | | | |
| ** The potassium originates from potassium citrate and KOH used for pH adjustment | | | | | | |
| **** energy contribution fibers not taken into account | | | | | | |

**Table 1C : Including an optional milk protein**

| **Ingredient** | **Ex. C1** | **Ex. C2** | **Ex. C3** | **Ex. C4** |
|---|---|---|---|---|
| **Energy content** (kcal/100 ml) | **400** | **400** | **400** | **400** |
| **Fat** (g/100 ml) | **38.7** | **40.3** | **40.4** | **40.3** |
| Blend of rapeseed/sunflower high oleic | 38.7 | 40.3 | 39.1 | 37.6 |
| Soy lecithin (emulsifier) | - | - | 1.3 | - |
| Fish oil | - | - | - | 2.7 |
| **Protein** (g/100 ml) | **8** | **5** | **5** | **5** |
| Non-micellar casein | 2.4 | 4.0 | 1.4 | 4.0 |
| Na-caseinate | 2.4 | 2.7 | 1.4 | 2.7 |
| K-caseinate | - | 1.3 | - | 1.3 |
| Micellar casein (MCI) | - | - | 3.4 | - |
| Whey protein hydrolysate (WPH) | 5.6 | 1.0 | 0.2 | 1.0 |
| **Carbohydrate** (g/100 ml) | **4.80** | **4.30** | **4.00** | **4.30** |
| sugars | 3.56 | 3.56 | 3.59 | 3.56 |
| sucrose | 3.49 | 3.49 | 3.49 | 3.49 |
| maltose | 0.05 | 0.05 | 0.05 | 0.05 |
| lactose | 0.02 | 0.02 | 0.05 | 0.02 |
| polysaccharides (CMC/MCC) | 0.07 | 0.07 | 0.07 | 0.07 |
| organic acids | 1.07 | 0.57 | 0.24 | 0.57 |
| others | 0.10 | 0.10 | 0.10 | 0.10 |

| **Heat stabiliser** (g/100 ml) | | | | |
|---|---|---|---|---|
| tripotassium citrate | 1.46 | 0.956 | - | 0.956 |
| monopotassium phosphate (KH₂PO₄) | - | - | 1.78 | - |

| **Minerals** (mg/100 ml) | | | | |
|---|---|---|---|---|
| Sodium | 99 | 59 | 65 | 59 |
| Potassium | 536 | 414 | 78 | 414 |
| Calcium | 222 | 222 | 223 | 222 |
| Phosphorous | 190 | 189 | 230 | 189 |
| Magnesium | 90 | 89 | 90 | 89 |
| Chloride | 38 | 59 | 67 | 59 |

**Table 1D : Including an optional vegetable protein**

| **Ingredient** | **Ex. D1** | **Ex. D2** |
|---|---|---|
| **Energy content** (kcal/100 ml) | **400** | **400** |
| **Fat** (g/100 ml) | **39.6** | **40.3** |
| Blend of rapeseed/sunflower high oleic | 39.6 | 40.3 |
| **Protein** (g/100 ml) | **6** | **6** |
| Non-micellar casein (Na-caseinate) | 2.7 | 2.7 |
| Pea protein hydrolysate | 3.3 | - |
| Soy protein hydrolysate | - | 3.3 |
| **Carbohydrate** (g/100 ml) | **5.99** | **4.50** |
| sugars | 4.99 | 3.50 |
| sucrose | 4.98 | 3.49 |
| maltose | 0.00 | 0.00 |
| lactose | 0.01 | 0.01 |
| polysaccharides (CMC/MCC) | 0.00 | 0.00 |
| organic acids | 1.00 | 1.00 |
| others | 0.00 | 0.00 |

| **Heat stabiliser** (g/100 ml) | | |
|---|---|---|
| tripotassium citrate | 1.45 | 1.45 |

| **Minerals** (mg/100 ml) | | |
|---|---|---|
| Sodium | 99 | 59 |
| Potassium | 536 | 414 |
| Calcium | 222 | 222 |
| Phosphorous | 190 | 189 |
| Magnesium | 90 | 89 |
| Chloride | 38 | 59 |

**Table 2 : Typical Package Trace Elements, vitamins and other additives**

| **Trace elements** | |
|---|---|
| Iron | 3.0 mg |
| Zinc | 3.4 mg |
| Selenium | 23 µg |
| Copper | 360 µg |
| Manganese | 0.7 mg |
| Chromium | 11 µg |
| Iodine | 45 µg |
| Molybdenum | 20 µg |

| **Vitamins** | |
|---|---|
| Vitamin A | 210 µg-RE |
| Vitamin D3 | 3 µg |
| Vitamin E | 6.04 mg-α-TE |
| Vitamin K | 31 µg |
| Thiamine hydrochloride | 0.4 mg |
| Riboflavin | 0.4 mg |
| Niacin | 5.1 mg-NE |
| Panthothenic acid | 1.5 mg |
| Vitamin B6 | 0.55 mg |
| Folic acid | 105 µg |
| Vitamin B 12 | 0.8 µg |
| Biotin | 16 µg |
| Vitamin C | 30 mg |

| **Others** | |
|---|---|
| Choline | 100 mg |
| Myoinositol | 11 mg |

## Claims

1. Shelf-stable liquid aqueous nutritional composition comprising non-micellar casein and more than 30 g fat per 100 ml of said composition, wherein the composition further comprises a heat stabilisation system.

2. Composition according to claim 1, having a fat content between 35 and 50 g per 100 ml of said composition.

3. Composition according to any one of the preceding claims, having a fat content of about 40 g per 100 ml of said composition.

4. Composition according to any one of the preceding claims, wherein the non-micellar casein content is at least 1 g per 100 ml of said composition.

5. Composition according to any one of the preceding claims, wherein the nonmicellar casein content ranges between 4 and 10 g per 100 ml of said composition.

6. Composition according to any one of the preceding claims, wherein the non-micellar casein is selected from the group of sodium caseinate, potassium caseinate or a mixture thereof.

7. Composition according to any one of the preceding claims, having a fat content of about 40 g per 100 ml of said composition and a non-micellar casein content of about 5 g per 100 ml of said composition.

8. Composition according to any of the preceding claims, further comprising a second protein other than non-micellar casein.

9. Composition according to claim 8, wherein the second protein is selected from the group of intact whey protein, whey protein hydrolysate (WPH), calcium caseinate, micellar casein (MCI) and mixtures thereof.

10. Composition according to any one of the preceding claims, wherein the heat stabilisation system is selected from the group of potassium citrate, potassium dihydrogen phosphate or mixtures thereof.

11. Composition according to claim 10, wherein the heat stabilisation system mainly comprises potassium citrate in case the second protein mainly comprises micellar casein.

12. Composition according to claim 10, wherein the heat stabilisation system mainly comprises potassium dihydrogen phosphate in case the second protein comprises whey protein hydrolysate.

13. Composition according to any one of the preceding claims, further having a digestible carbohydrate content ranging between 1 and 10 g per 100 ml of said composition, preferably equal to about 5 g per 100 ml of said composition.

14. Composition according to any one of the preceding claims, further comprising vitamins, minerals, and non-digestible carbohydrates such as prebiotics.

15. Composition according to any one of the preceding claims, having an energy content of at least 400 kcal/100 ml.

16. Composition according to claim 1, comprising 4 g non-micellar casein per 100 ml of said composition, potassium citrate as a heat stabilisation system, and 40.3 g fat per 100 ml of said composition.

17. Composition according to claim 16, further comprising 1 g of whey protein hydrolysate, and optionally 4.3 g of carbohydrates per 100 ml of said composition.

18. Composition according to any one of the preceding claims, further comprising an acid oligosaccharide with a DP between 2 and 250, prepared from pectin, alginate and mixtures thereof; and a neutral oligosaccharide, selected from the group of fructans, fructooligosaccharides, indigestible dextrins, galactooligosaccharides including transgalactooligosaccharides, xylooligosaccharides, arabinooligosaccharides, glucooligosaccharides, mannooligosaccharides, fucooligosaccharides, and mixtures thereof.

19. Composition according to any one of the preceding claims, further comprising a mixture of low viscosity pectin with an average DP of 2 to 250, a galactose-based neutral oligosaccharide and a long chain fructose-based oligosaccharide with an average DP of 10 to 60.

20. Method for the production of a composition according to any one of the preceding claims, comprising the steps of :
(i) preparing an aqueous composition by dissolving into water the casein source and the emulsifier, a heat stabilisation system, and optionally other ingredients such as a precipitation stabiliser, other proteins, carbohydrates and minerals ;
(ii) adjusting the pH to between 6.5 and 7.2 ;
(iii) adding the fat source to the composition obtained by step (ii) ;
(iv) homogenising the composition obtained by step (iii) and subjecting it to one or more heat treatments.

21. Composition according to any one of claims 1 to 19 for use in the management of conditions in malnourished persons or persons at risk of becoming malnourished, and which are in need of liquid oral nutrition.

22. Composition according to claim 21, for use in the treatment of malnourished persons, persons at risk of malnourishment, metabolically stressed people, and elderly.

## Patentansprüche

1. Haltbare flüssige wässrige Nährstoffzusammensetzung, umfassend nicht-micellares Casein und mehr als 30 g Fett pro 100 ml der Zusammensetzung, wobei die Zusammensetzung ferner ein Wärmestabilisierungssystem umfasst.

2. Zusammensetzung nach Anspruch 1, die einen Fettgehalt von zwischen 35 und 50 g pro 100 ml der Zusammensetzung aufweist.

3. Zusammensetzung nach einem der vorhergehenden Ansprüche, die einen Fettgehalt von etwa 40 g pro 100 ml der Zusammensetzung aufweist.

4. Zusammensetzung nach einem der vorhergehenden Ansprüche, wobei der nichtmicellare Caseingehalt mindestens 1 g pro 100 ml der Zusammensetzung beträgt.

5. Zusammensetzung nach einem der vorhergehenden Ansprüche, wobei der nichtmicellare Caseingehalt zwischen 4 g und 10 g pro 100 ml der Zusammensetzung beträgt.

6. Zusammensetzung nach einem der vorhergehenden Ansprüche, wobei das nichtmicellare Casein ausgewählt ist aus der Gruppe aus Natriumcaseinat, Kaliumcaseinat oder einer Mischung davon.

7. Zusammensetzung nach einem der vorhergehenden Ansprüche, die einen Fettgehalt von etwa 40 g pro 100 ml der Zusammensetzung und einen nichtmicellaren Caseingehalt von etwa 5 g pro 100 ml der Zusammensetzung aufweist.

8. Zusammensetzung nach einem der vorhergehenden Ansprüche, ferner umfassend ein zweites Protein mit Ausnahme von nicht-micellarem Casein.

9. Zusammensetzung nach Anspruch 8, wobei das zweite Protein ausgewählt ist aus der Gruppe aus intaktem Molkenprotein, Molkenprotein-Hydrolysat, Calciumcaseinat, micellarem Casein und Mischungen davon.

10. Zusammensetzung nach einem der vorhergehenden Ansprüche, wobei das Wärmestabilisierungssystem ausgewählt ist aus der Gruppe aus Kaliumcitrat, Kaliumdihydrogenphosphat oder Mischungen davon.

11. Zusammensetzung nach Anspruch 10, wobei das Wärmestabilisierungssystem im Wesentlichen Kaliumcitrat umfasst, sofern das zweite Protein im Wesentlichen micellares Casein umfasst.

12. Zusammensetzung nach Anspruch 10, wobei das Wärmestabilisierungssystem im Wesentlichen Kaliumdihydrogenphosphat umfasst, sofern das zweite Protein im Wesentlichen Molkenprotein-Hydrolysat umfasst.

13. Zusammensetzung nach einem der vorhergehenden Ansprüche, die ferner einen verwertbaren Kohlenhydratanteil zwischen 1 und 10 g pro 100 ml der Zusammensetzung, vorzugsweise etwa 5 g pro 100 ml der Zusammensetzung, aufweist.

14. Zusammensetzung nach einem der vorhergehenden Ansprüche, ferner umfassend Vitamine, Mineralien und nicht-verwertbare Kohlenhydrate wie Präbiotika.

15. Zusammensetzung nach einem der vorhergehenden Ansprüche, die einen Energiegehalt von mindestens 400 kcal/100 ml aufweist.

16. Zusammensetzung nach Anspruch 1, umfassend 4 g nicht-micellares Casein pro 100 ml der Zusammensetzung, Kaliumcitrat als Wärmestabilisierungssystem und 40,3 g Fett pro 100 ml der Zusammensetzung.

17. Zusammensetzung nach Anspruch 16, ferner umfassend 1 g Molkenprotein-Hydrolysat und optional 4,3 g Kohlenhydrate pro 100 ml der Zusammensetzung.

18. Zusammensetzung nach einem der vorhergehenden Ansprüche, ferner umfassend ein saures Oligosaccharid mit einem Polymerisationsgrad zwischen 2 und 250, hergestellt aus Pektin, Alginat und Mischungen davon und ein neutrales Oligosaccharid, ausgewählt aus der Gruppe aus Fruktanen, Fructooligosacchariden, nicht-verwertbaren Dextrinen, Galactooligosacchariden einschließlich Transgalactooligosacchariden, Xylooligosacchariden, Arabinooligosacchariden, Glucooligosacchariden, Mannooligosacchariden, Fucooligosacchariden und Mischungen davon.

19. Zusammensetzung nach einem der vorhergehenden Ansprüche, ferner umfassend eine Mischung von niedrig viskosem Pektin mit einem mittleren Polymerisationsgrad von 2 bis 250, einem Galactose-basierten neutralen Oligosaccharid und einem langkettigen Fructose-basierten Oligosaccharid mit einem mittleren Polymerisationsgrad von 10 bis 60.

20. Verfahren zur Herstellung einer Zusammensetzung nach einem der vorhergehenden Ansprüche, umfassend die Schritte:
(i) Bereitstellen einer wässrigen Zusammensetzung durch Lösen der Caseinquelle, des Emulgators, ein Wärmestabilisierungssystem und optional weiterer Inhaltsstoffe, wie ein Fällungsstabilisator, andere Proteine, Kohlenhydrate und Mineralien, in Wasser;
(ii) Einstellen des pH-Werts auf zwischen 6,5 und 7,2;
(iii) Zugeben der Fettquelle zu der in Schritt (ii) erhaltenen Zusammensetzung;
(iv) Homogenisieren der in Schritt (iii) erhaltenen Zusammensetzung und eine oder mehrere Wärmebehandlungen derselben.

21. Verwendung der Zusammensetzung nach einem der Ansprüche 1 bis 19 zur Handhabung von Zuständen bei unterernährten Personen oder Personen mit Risiko zur Unterernährung, die orale Flüssignahrung benötigen.

22. Verwendung der Zusammensetzung nach Anspruch 21 zur Behandlung von unterernährten Personen, Personen mit Risiko zur Unterernährung, metabolisch gestressten Personen und Senioren.

## Revendications

1. Composition nutritionnelle aqueuse liquide de longue conservation comprenant de la caséine non micellaire et plus de 30 g de graisse pour 100 mL de ladite composition, dans laquelle la composition comprend en outre un système de stabilisation à la chaleur.

2. Composition selon la revendication 1, ayant une teneur en graisse entre 35 et 50 g pour 100 mL de ladite composition.

3. Composition selon l'une quelconque des revendications précédentes, ayant une teneur en graisse d'environ 40 g pour 100 mL de ladite composition.

4. Composition selon l'une quelconque des revendications précédentes, dans laquelle la teneur en caséine non micellaire est d'au moins 1 g pour 100 mL de ladite composition.

5. Composition selon l'une quelconque des revendications précédentes, dans laquelle la teneur en caséine non micellaire varie entre 4 et 10 g pour 100 mL de ladite composition.

6. Composition selon l'une quelconque des revendications précédentes, dans laquelle la caséine non micellaire est choisie dans le groupe du caséinate de sodium, du caséinate de potassium ou de l'un de leurs mélanges.

7. Composition selon l'une quelconque des revendications précédentes, ayant une teneur en graisse d'environ 40 g pour 100 mL de ladite composition et une teneur en caséine non micellaire d'environ 5 g pour 100 mL de ladite composition.

8. Composition selon l'une quelconque des revendications précédentes, comprenant en outre une seconde protéine autre que la caséine non micellaire.

9. Composition selon la revendication 8, dans laquelle la seconde protéine est choisie dans le groupe de la protéine de lactosérum intacte, de l'hydrolysat de protéine de lactosérum (WPH), du caséinate de calcium, de la caséine micellaire (MCI) et de leurs mélanges.

10. Composition selon l'une quelconque des revendications précédentes, dans laquelle le système de stabilisation à la chaleur est choisi dans le groupe du citrate de potassium, du dihydrogénophosphate de potassium ou de leurs mélanges.

11. Composition selon la revendication 10, dans laquelle le système de stabilisation à la chaleur comprend principalement du citrate de potassium dans le cas où la seconde protéine comprend principalement de la caséine micellaire.

12. Composition selon la revendication 10, dans laquelle le système de stabilisation à la chaleur comprend principalement du dihydrogénophosphate de potassium dans le cas où la seconde protéine comprend de l'hydrolysat de protéine de lactosérum.

13. Composition selon l'une quelconque des revendications précédentes, ayant en outre une teneur en glucide digestible variant entre 1 et 10 g pour 100 mL de ladite composition, de préférence égale à environ 5 g pour 100 mL de ladite composition.

14. Composition selon l'une quelconque des revendications précédentes, comprenant en outre des vitamines, des minéraux et des glucides non digestibles tels que des prébiotiques.

15. Composition selon l'une quelconque des revendications précédentes, ayant une teneur en énergie d'au moins 400 kcal/100 mL.

16. Composition selon la revendication 1, comprenant 4 g de caséine non micellaire pour 100 mL de ladite composition, du citrate de potassium en tant que système de stabilisation à la chaleur et 40,3 g de graisse pour 100 mL de ladite composition.

17. Composition selon la revendication 16, comprenant en outre 1 g d'hydrolysat de protéine de lactosérum et facultativement 4,3 g de glucides pour 100 mL de ladite composition.

18. Composition selon l'une quelconque des revendications précédentes, comprenant en outre un oligosaccharide acide avec un DP entre 2 et 250, préparé à partir de pectine, d'alginate et de leurs mélanges ; et un oligosaccharide neutre, choisi dans le groupe des fructanes, fructo-oligosaccharides, dextrines indigestibles, galacto-oligosaccharides, dont les transgalacto-oligosaccharides, xylo-oligosaccharides, arabino-oligosaccharides, gluco-oligosaccharides, manno-oligosaccharides, fuco-oligosaccharides, et leurs mélanges.

19. Composition selon l'une quelconque des revendications précédentes, comprenant en outre un mélange de pectine de basse viscosité avec un DP moyen de 2 à 250, un oligosaccharide neutre à base de galactose et un oligosaccharide à base de fructose à longue chaîne avec un DP moyen de 10 à 60.

20. Méthode de production d'une composition selon l'une quelconque des revendications précédentes, comprenant les étapes consistant à :
(i) préparer une composition aqueuse en dissolvant dans de l'eau la source de caséine et l'émulsifiant, un système de stabilisation à la chaleur, et facultativement d'autres ingrédients tels qu'un stabilisant de précipitation, d'autres protéines, glucides et minéraux ;
(ii) ajuster le pH entre 6,5 et 7,2 ;
(iii) ajouter la source de graisse à la composition obtenue par l'étape (ii) ;
(iv) homogénéiser la composition obtenue par l'étape (iii) et la soumettre à un ou plusieurs traitements à la chaleur.

21. Composition selon l'une quelconque des revendications 1 à 19, pour son utilisation dans la gestion de conditions de personnes malnutries ou de personnes présentant un risque de devenir malnutries, et qui sont en besoin d'une nutrition orale liquide.

22. Composition selon la revendication 21, pour son utilisation dans le traitement de personnes malnutries, de personnes présentant un risque de malnutrition, de personnes métaboliquement stressées et de personnes âgées.
